# EUROPEAN PATENT APPLICATION

(11) **EP 4 708 315 A1**
(43) Date of publication of application: **11.03.2026**
(21) Application number: 25195158.8
(22) Date of filing: 11.08.2025
(51) Int. Cl.: G16H 30/40, G16H 40/40, G16H 40/63, G16H 50/20

(54) **ANATOMICAL LOCATION TAGGING OF PHYSIOLOGICAL PARAMETERS**

(30) Priority: 04.09.2024 US 202418824821
(71) Applicant: GE Precision Healthcare LLC, Waukesha, WI 53188 (US)
(72) Inventor: MISHRA, Isani, 560066 Bengaluru (IN); RAGHAVAN, Jayaram, 560066 Bengaluru (IN)
(74) Representative: Kilburn & Strode LLP

(57) **Abstract**

Systems and methods are disclosed for tagging anatomical locations of monitoring devices on a patient in a reference image on an application. The application excludes data from the monitoring devices from analysis by a clinical decision support system when the monitoring devices are misplaced, for example in locations other than the tagged anatomical locations.

## Description

### TECHNICAL FIELD

The present description relates generally to anatomical location tagging of physiological parameters measured by monitoring devices on a patient.

### BACKGROUND AND SUMMARY

Sensors and other monitoring devices may produce different physiological data measurements (e.g., blood oxygen saturation, respiratory rate, temperature, blood pressure, etc.) when placed in different anatomical locations of a patient. Comparing physiological data at different anatomical locations may aid clinicians in diagnosing medical conditions. For example, a blood pressure difference between right and left arms may indicate cardiovascular problems. For another example, specific magnitudes of blood oxygen saturation differences between right arm and right foot may indicate critical congenital heart disease.

Clinical decision support (CDS) systems may be used by clinicians to analyze patient data, for example physiological data gathered by such monitoring devices. The CDS systems may take into account anatomical locations of the sensors when running algorithms, such as diagnostic algorithms based upon physiological data gathered by monitoring devices. Therefore, placement of monitoring devices at incorrect anatomical locations on a patient may decrease accuracy of a diagnosis produced by the CDS system. Using the example provided above, if the CDS runs an algorithm for diagnosing congenital heart disease by comparing blood oxygen saturation in the right arm and right foot, and the blood oxygen saturation monitoring devices are placed in locations other than right arm and right foot (e.g., left arm and right foot), incorrect diagnosis may result from the inaccurate data.

Previous methods to ensure correct placement of monitoring devices include color coding the probes with respect to anatomical location and using signal quality indicators. Such methods are at least sometimes inadequate in ensuring correct anatomical location of monitoring devices throughout the duration patients are monitored by the monitoring devices. For example, the clinician may not be able to monitor the patient's physiological data and view monitoring device locations remotely with such methods. Further, such methods may not automatically affect CDS systems. For example, such methods may not indicate the anatomical location being input to the CDS system, but rather assume the locations for given protocols or diagnostic algorithms. Therefore, outputs of the CDS systems may be based upon inaccurate data gathered when the monitoring devices are misplaced and thus the outputs may not reflect the patient's actual condition, but rather a condition misrepresented by the inaccurate data.

Thus, embodiments are disclosed herein that solve at least some of the issues described above with instructions stored in memory of a user device that when executed: receive a reference image of a monitoring device adapted to measure physiological data of a patient and tagged to an anatomical location of the patient; provide the tagged anatomical location to a CDS system; and in response to the CDS system detecting that the monitoring device is misplaced (e.g., by detecting degradation or loss of signal), displaying an alert that the monitoring device is misplaced. The reference image may be inputted to the user device by a clinician following positioning of the monitoring devices on the selected anatomical locations of the patient. The reference image may include a photograph or abstracted physical representation of the patient (e.g., cartoon corresponding to age and gender of the patient) and the monitoring devices at the tagged anatomical locations. The clinician may view the alert and be aware that at least one of one or more monitoring devices is misplaced and the CDS system is no longer receiving data therefrom. In this way, inaccurate data gathered by monitoring devices being positioned in locations other than the tagged anatomical locations in the reference image may not be consumed by the CDS system. As such, accuracy of an output of the CDS system may be increased. Further, the clinician may view placement of the monitoring devices and the physiological data gathered remotely on the user device such that statuses of the patient and the monitoring devices may be monitored by the clinician from locations other than physically with the patient. Increased ease of access to patient physiological data and monitoring device placement may further increase quality and speed of clinical decisions.

It should be understood that the summary above is provided to introduce in simplified form a selection of concepts that are further described in the detailed description. It is not meant to identify key or essential features of the claimed subject matter, the scope of which is defined uniquely by the claims that follow the detailed description. Furthermore, the claimed subject matter is not limited to implementations that solve any disadvantages noted above or in any part of this disclosure.

### BRIEF DESCRIPTION OF THE FIGURES

FIG. 1 schematically shows a patient monitoring system.
FIG. 2 schematically shows the patient monitoring system, including a device with an application of the present disclosure, when the monitoring devices are positioned correctly.
FIG. 3 schematically shows the patient monitoring system when monitoring devices are positioned incorrectly.
FIG. 4 shows a flowchart of a method for a clinician monitoring a patient using the device.
FIG. 5 shows a flowchart of a method for operating the application.
FIG. 6 schematically shows a patient monitoring system.
FIG. 7 shows an example of a display screen on a user interface of the device.
FIG. 8 shows another example of a display screen on the user interface of the device.
FIG. 9 shows another example of a display screen on the user interface of the device.
FIG. 10 shows an example of a reference image.
FIG. 11 shows a signal diagram for a patient monitoring system.

### DETAILED DESCRIPTION

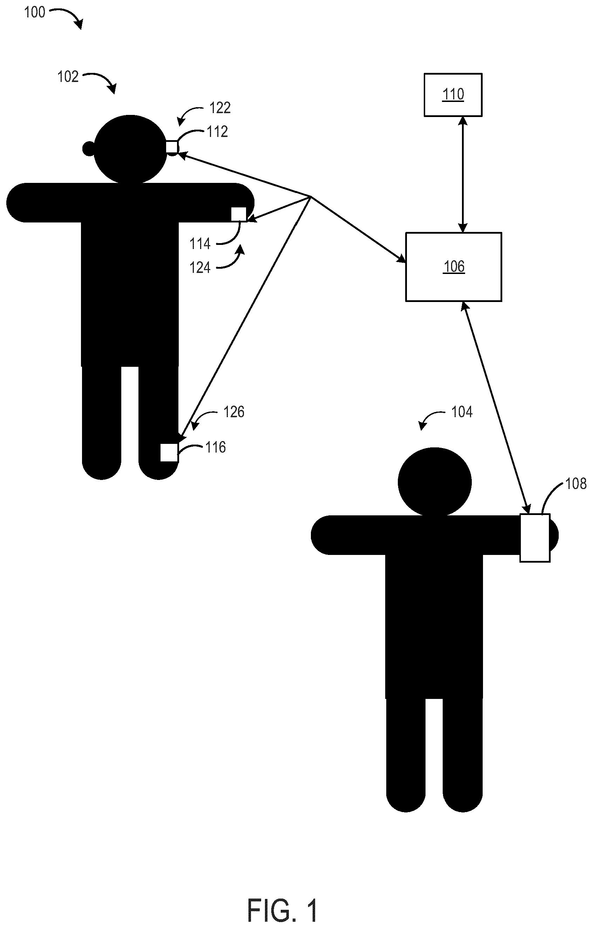

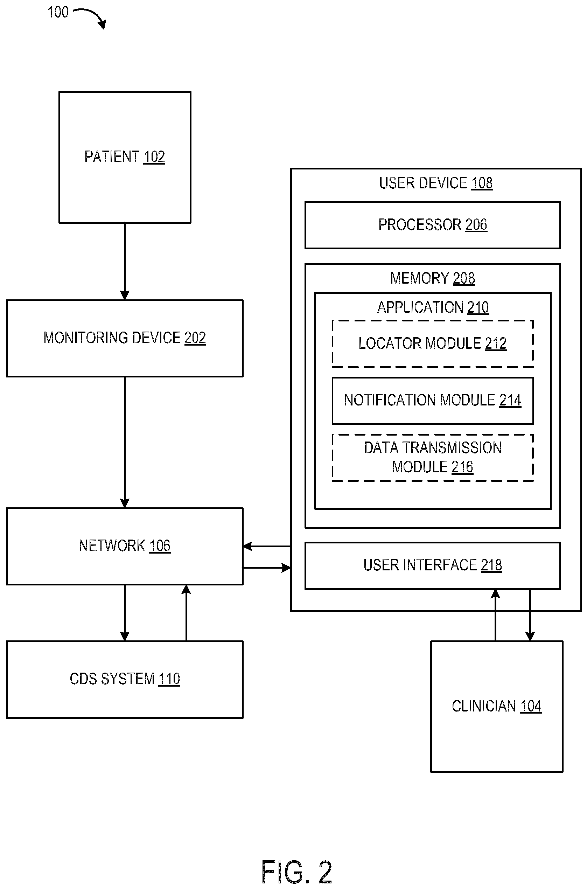

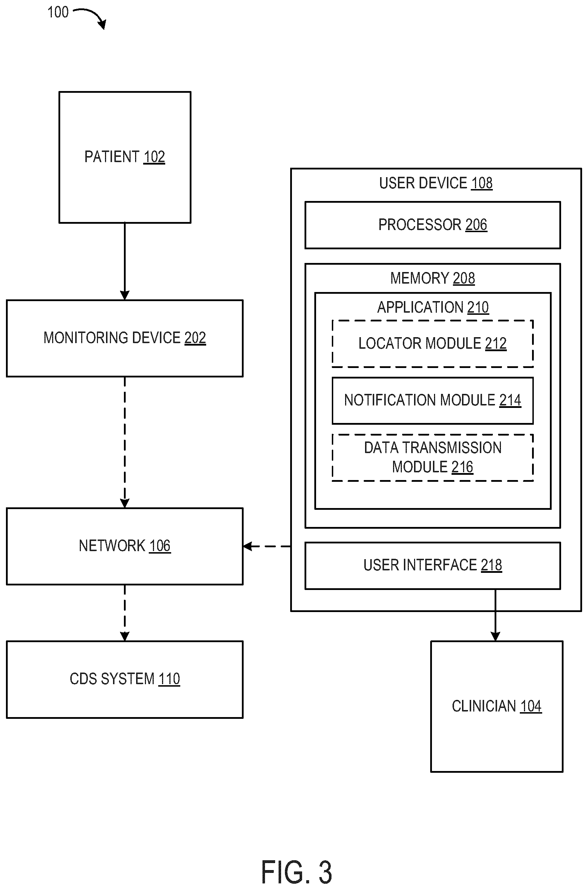

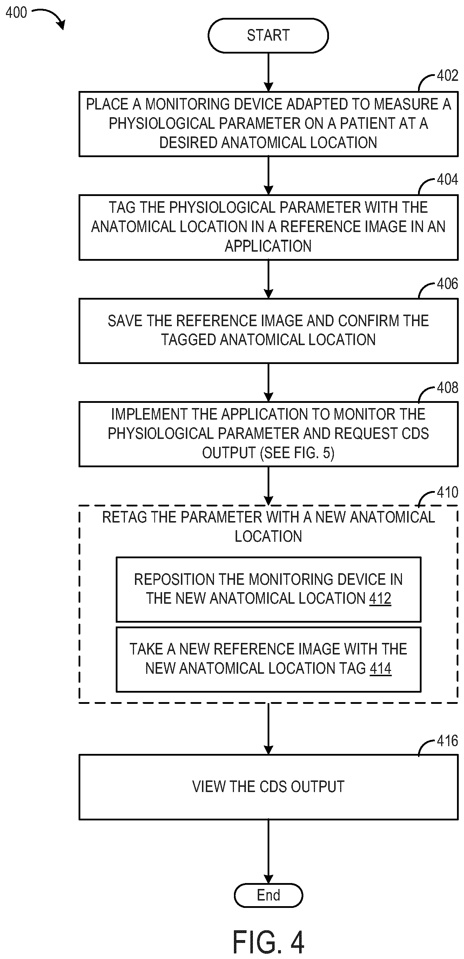

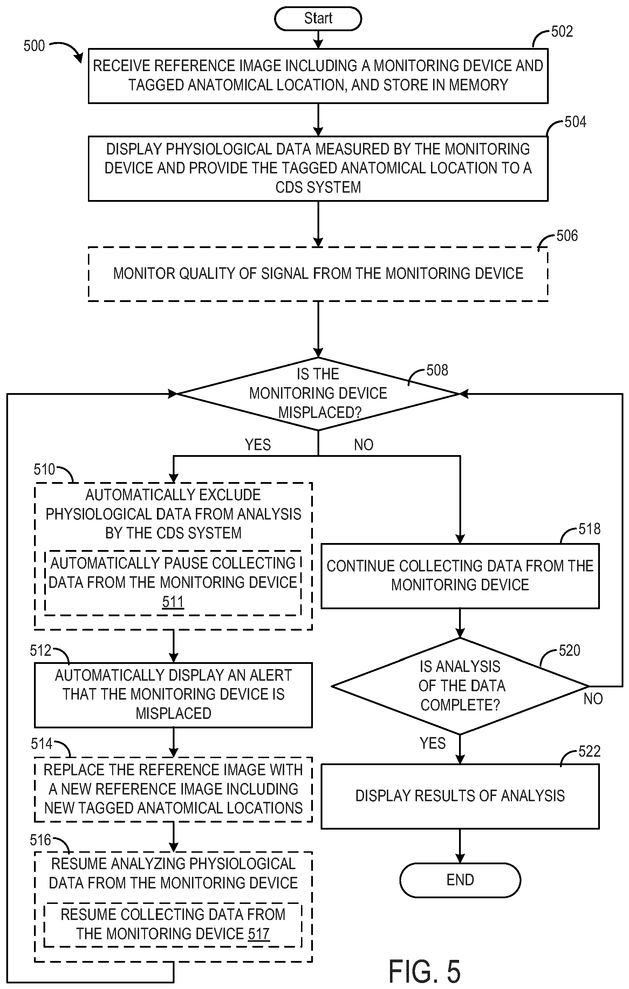

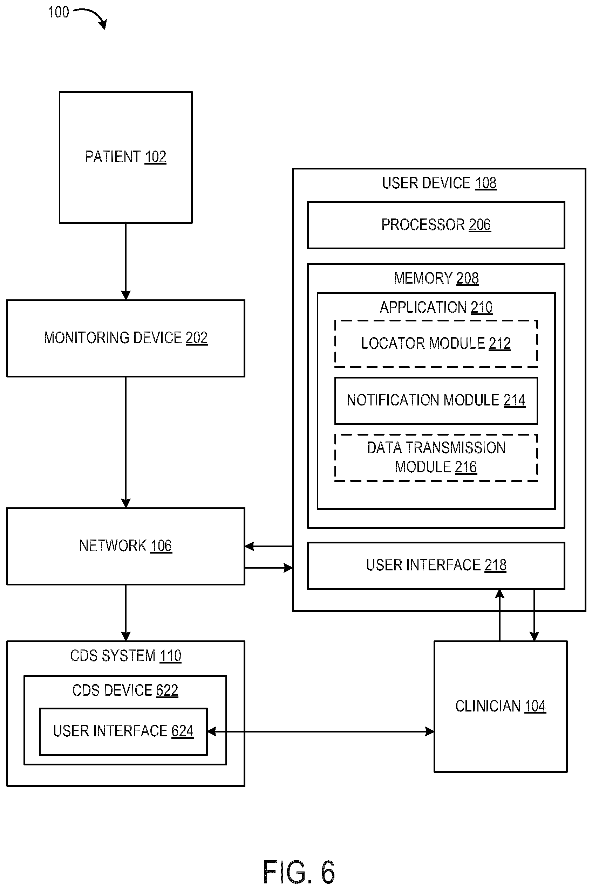

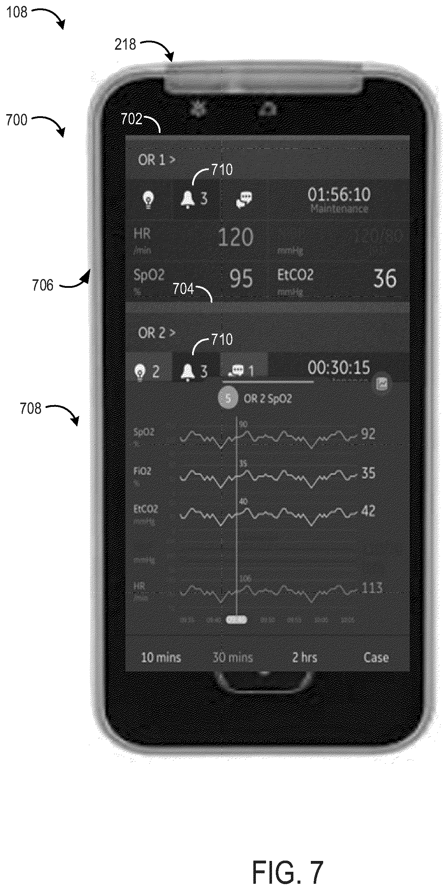

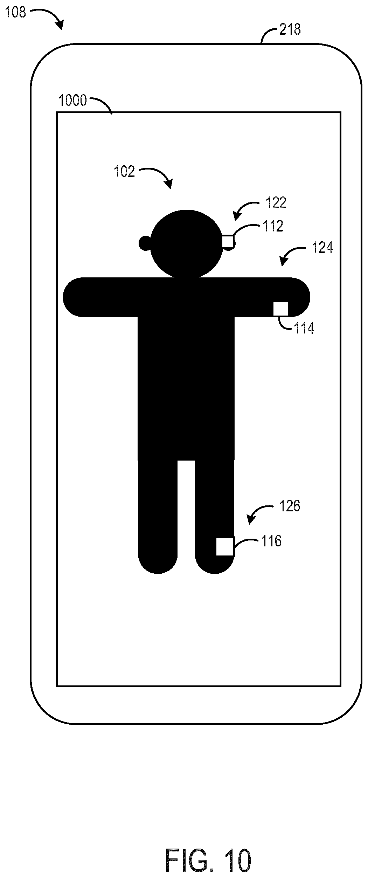

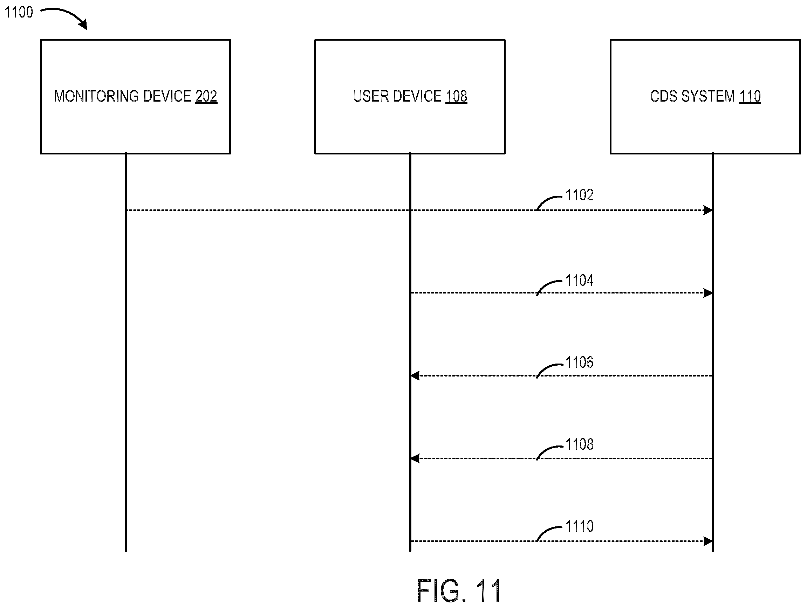

The following description relates to systems and methods for anatomical location tagging of physiological parameters during monitoring of a patient by a clinician. The tagging may increase accuracy of data gathered on the physiological parameters, thereby increasing likelihood of effective clinical decisions (e.g., correct diagnosis) by the clinician with aid from a clinical decision support (CDS) system that consumes the data and provides a suggested clinical decision. A patient monitoring system is shown schematically in FIG. 1, including a patient being monitored by a clinician using a user device having an application stored in memory thereof with instructions executable to respond to misplacement of monitoring devices from positions pre-selected and verified by the clinician in a reference image. The reference image may be a photograph or abstracted physical representation (e.g., abstracted cartoon representation that indicates age and gender of the patient) of the patient and the monitoring devices. The reference image may include tagged anatomical locations for each physiological parameter measured by the monitoring devices. An exemplary reference image is shown in FIG. 10. The application may communicate with the CDS system, display data in real-time, including the tagged anatomical locations, and alert the clinician of signal quality changes through a method shown in a flowchart in FIG. 5. For example, when the monitoring devices are positioned correctly (e.g., the same as in the reference image), the monitoring devices may transmit data to the CDS system, as shown schematically in FIG. 2. In some examples, when one or more of the monitoring devices are not positioned correctly, causing degradation to signal quality or loss of the signal, the application may pause transmission of data from the monitoring devices to the CDS system or otherwise exclude the data from analysis by the CDs system and alert the clinician, as shown schematically in FIG. 3. Another patient monitoring system example is shown schematically in FIG. 6. A signal diagram showing communication between the monitoring devices, the CDS system, and the user device is shown in FIG. 11. The clinician may interact with the application, including inputting a reference image, replacing the reference image, and viewing data from the monitoring devices, via a user interface of the device by a method shown in a flowchart in FIG. 4. Examples of display screens provided to the clinician on the input device are shown in FIGS. 7-9. By tagging anatomical locations of the patient with physiological parameters measured by monitoring devices positioned at those locations, the clinician may signal to the CDS system that the monitoring devices are repositioned correctly, where previous systems may not allow for such confirmation. Additionally, in examples where the user device automatically pauses data collection or otherwise excludes data from analysis when the monitoring devices are misplaced at other locations, accuracy of the data consumed by the CDS system may be increased. Accuracy of the data may be increased because data gathered from misplaced monitoring devices (e.g., that are not in the tagged anatomical locations) is not included in response to monitoring devices being misplaced. Misplaced monitoring devices may measure physiological data that differs from a state of the intended anatomical location and may misrepresent a state of the patient. For example, blood pressure of a patient that has undergone surgery on the right arm may be measured differently when the blood pressure monitoring devices is positioned on the right arm vs the left arm of the patient. Therefore, algorithms that take physiological data as well as corresponding anatomical locations as inputs may produce more accurate outputs when data with mismatched data and locations is omitted by automatically pausing data consumption or otherwise excluding such mismatched data from analysis with the algorithms. In this way, clinical decisions recommended by the CDS system may be made based upon more accurate information, and therefore a likelihood of incorrect clinical decision recommendations according to the condition of the patient may be reduced.

The technical solution provided by the disclosed systems and methods improves the functioning of CDS systems by ensuring the accuracy and reliability of the physiological data inputs. Prior systems were in some cases susceptible to providing inaccurate data to CDS algorithms when monitoring devices were misplaced on the patient, leading to incorrect clinical decision recommendations.

The present disclosure at least partially addresses this technical problem through the use of a reference image that tags the monitoring devices to specific anatomical locations on the patient. By monitoring the signal quality of the monitoring devices and reacting when misplacement is detected to exclude data from misplaced monitoring devices, the system may prevent the CDS from consuming inaccurate data. This increases the accuracy and reliability of the CDS outputs, leading to more informed clinical decisions and increasing quality of patient outcomes.

Furthermore, in applicable examples, the automatic pausing of data transmission in response to monitoring device misplacement may reduce unnecessary computational load on the CDS system, improving its efficiency and reducing resource demands. This technical improvement allows the CDS system to focus its processing power on analyzing accurate physiological data, rather than wasting resources on inaccurate data from misplaced monitoring devices.

The disclosed systems and methods provide a technical solution to the problem of ensuring accurate physiological data inputs to clinical decision support systems, thereby improving the functioning of those systems and leading to better informed clinical decisions. The approach is specifically directed to the real-world issues surrounding particular hardware used in a particular way with regards to anatomical location tagging of physiological parameters measured by monitoring devices on a patient, as opposed to generic sensors and generic sensor data processed by generic computer hardware. The automatic detection and exclusion of data (e.g., by pausing data transmission), particularly physiological parameters of a patient measured by healthcare monitoring devices in the specifics of a healthcare setting, from misplaced monitoring devices in CDS analysis is a specific technological improvement over prior systems that lacked such capabilities.

It is to be understood that the systems illustrated in the attached drawings, and described in the following specification are exemplary embodiments of the inventive concepts defined herein. For purposes of discussion, the drawings are described collectively. Thus, like elements may be commonly referred to herein with like reference numerals and may not be reintroduced.

Turning to FIG. 1, a patient monitoring system 100 is schematically shown. The patient monitoring system 100 includes a patient 102, a clinician 104, and a user device 108.

One or more monitoring devices adapted to monitor physiological parameters may be positioned on the patient 102 by the clinician 104. For example, a first monitoring device 112 may be positioned at a first anatomical location 122, a second monitoring device 114 may be positioned at a second anatomical location 124, and a third monitoring device 116 may be positioned at a third anatomical location 126. The monitoring devices 112, 114, 116 may measure physiological parameters such as blood pressure, blood oxygen level, heart rate, ankle brachial index, temperatures, and the like. For example, the anatomical locations 122, 124, 126 are shown respectively at the left ear, left arm, and the left leg, however, the anatomical locations may be any appropriate locations on the body of the patient 102 according to a desired physiological parameter measurement selected by the clinician 104. The monitoring devices 112, 114, 116 may produce different measurements when placed at different anatomical locations. For example, the monitoring device 112 may generate a higher or lower physiological parameter reading when positioned at the first anatomical location 122 compared to when positioned at the second anatomical location 124. Comparison of a physiological parameter measured at different anatomical locations may be used in reaching clinical decisions. For example, two or more of the monitoring devices 112, 114, and 116 may measure the same physiological parameter and a difference, ratio, or other metric in data from the two or more monitoring devices falling within a range may indicate a diagnosis of the patient 102, such as is used in determining an ankle brachial index. Additionally, positioning of an individual monitoring device may affect accuracy of the measurement produced by the monitoring device. For example, one type of monitoring device (e.g., oximeter) may be less accurate when located on a finger than an ear. In such an example, the inaccurate physiological data gathered by the monitoring device being placed on the finger may result in an inaccurate diagnosis, and therefore unsuitable treatment. Thus, positioning of the monitoring devices 112, 114, 116 on the patient 102 may affect accuracy of a diagnosis or other clinical decision.

Such clinical decisions regarding the patient 102 may be made by the clinician 104 by directly assessing data gathered by the monitoring devices 112, 114, 116. Additionally, the clinician 104 may utilize a clinical decision support (CDS) system 110. The CDS system 110 may include software including code that when executed performs algorithms to provide clinical decisions recommendations (e.g., diagnosis, treatment plan, etc.) as outputs to the clinician 104. The software may be deployed on a backend data platform repository accessible via a network 106. The network 106 may include any interconnected communication network (e.g., cellular communication network, local area network, wide area network, public network, enterprise private network, or a combination thereof) allowing communication between the monitoring devices 112, 114, 116, the user device 108, and the CDS system 110. The monitoring devices 112, 114, 116, the user device 108, and the CDS system 110 may connect to the network 106 via a hardwired link and/or a wireless link.

The CDS system 110 may take locations of the monitoring devices 112, 114, 116 as well as data gathered by the monitoring devices 112, 114, 116 as inputs. For example, a blood pressure difference greater than a threshold (e.g., 10-15 points) between the right and left arms may indicate cardiovascular problems. As such, the CDS system 110 may take monitoring device measurements and locations of the monitoring devices as inputs for algorithms that interpret the physiological parameter measurements and output suggested clinical decisions accordingly. The CDS system 110 may be uninformed in the case of misplacement of the monitoring devices in previous patient monitoring examples. As misplacement of the monitoring devices 112, 114, 116 may affect measurements of the physiological parameters of the patient 102, the CDS system 110 may consume inaccurate data when monitoring devices 112, 114, 116 are misplaced. Misplacement of the monitoring devices 112, 114, 116 may include the monitoring devices 112, 114, 116 being located in a position other than the respective anatomical locations 122, 124, 126 chosen by the clinician 104. Therefore, the CDS system 110 may produce clinical decision recommendations to the clinician based on misrepresented data (e.g., data from undesired anatomical locations) when the monitoring devices 112, 114, 116 are positioned incorrectly (e.g., not at the desired anatomical locations selected by the clinician).

To ensure correct placement of the monitoring devices 112, 114, 116 and prevent inaccurate decision recommendations from the CDS system 110, the clinician 104 may utilize a user device 108 with an application stored in memory thereof configured to reflect current status of data flow from the monitoring devices 112, 114, 116 to the CDS system 110 and alert the clinician 104 of misplacement of the monitoring devices 112, 114, 116. In some examples, data gathered from misplaced monitoring devices 112, 114, 116 may be labeled in such a way where the CDS system is informed the data is to be excluded from analysis. In some examples, the user device 108 may pause data transmission from the monitoring devices 112, 114, 116 to the CDS system 110. In this way, the application on the user device 108 may reduce (e.g., prevent) inaccurate clinical decision suggestions generated by the CDS system 110 from the monitoring devices 112, 114, 116 being misplaced. Further, in such examples where data transmission is paused, the application may reduce computing resource demand by halting data consumption by the CDS 110 in response to misplacement of the monitoring devices 112, 114, 116.

Turning to FIG. 2, the patient monitoring system 100 is shown schematically with arrows indicating communication pathways, including of physiological data gathered from monitoring devices 202, as well as inputs and outputs of both the user device 108 and the CDS system 110. Specifically, FIG. 2 shows the patient monitoring system 100 in a first state where monitoring devices 202 are positioned correctly on the patient 102 according to anatomical locations pre-selected by the clinician 104. The monitoring devices 202 may represent one or more monitoring devices adapted to measure physiological data and positioned by the clinician 104 on anatomical locations of the patient 102, such as the monitoring devices 112, 114, 116 positioned at the anatomical locations 122, 124, 126 in FIG. 1.

The anatomical locations selected by the clinician 104 may be input to the user device 108 via user interface 218. For example, the anatomical locations and types (e.g., according to the physiological parameter measured) of the monitoring devices 202 on the patient 102 may be tagged in a reference image that is uploaded to the user device 108 by the clinician 104 via the user interface 218. Turning briefly to FIG. 10, an example of a reference image 1000 is schematically depicted, where the reference image 1000 is displayed on the user interface 218 and stored in memory (e.g., memory 208 of FIG. 2) of the device 108. The reference image 1000 includes a physical representation of the patient 102 that includes the patient body, limbs, and head, as well as monitoring devices and their respective anatomical locations relative to the physical representation. For example, as described above, the first monitoring device 112 is tagged at the first anatomical location 122, the second monitoring device 114 is tagged at the second anatomical location 124, and the third monitoring device 116 is tagged at the third anatomical location 126. In some examples, the physical representation may be a physically correct photograph of the patient 102 taken by a camera of a device such as the device 108. In other examples, the physical representation may be an abstracted (e.g., cartoon) representation of the patient 102. An abstracted representation (e.g., cartoon indicating age and gender) may protect patient privacy by storing data pertaining to the monitoring device locations relative to the patient anatomy and no other visual information that would be available in a photograph. The monitoring devices may be represented by icons related to the physiological parameters measured, in at least some examples. The reference image 1000 may also include other features in addition to monitoring devices, in some examples. For example, indicators of prior surgical procedures may be tagged to anatomical locations where the procedure was performed. In this way, the clinician may ensure the positioning of the monitoring devices is appropriate considering the condition of the patient and past operations and treatments. For example, if a patient has undergone an operation on a breast or axilla, a blood pressure sensor may be placed on an arm on the opposite side of the patient. The reference image 1000 may provide a visual representation for the clinician to view placement of the monitoring devices 112, 114, 116 remotely (e.g., out of physical sight of the patient) such that the clinician may monitor the patient even while not physically with the patient or in direct visual range of the patient.

Returning to FIG. 2, as described above, the monitoring devices 202 may measure physiological data from the patient 102. The physiological data may include blood oxygen saturation, blood pressure, heart rate, any other health-related metric for a condition of the patient measureable by sensors and other monitoring devices, or a combination thereof. The monitoring devices 202 may send signals wirelessly to the user device 108 and/or the CDS system 110 via the network 106. The signals may include a data packet comprising physiological data measured by the monitoring devices 202 as well as a signal quality index, where the signal quality index is a metric for quality of the signal being transmitted from the monitoring devices 202. In at least some examples, the signals may be delivered to the CDS system 110 and subsequently provided to the user device 108 such that the user device 108 reflects status of data flow to the CDS system in real-time or near real-time. In other examples, the user device 108 may receive physiological data directly from the monitoring devices and provide the signals containing the physiological data to the CDS system 110 via the network 106.

The CDS system 110 may produce an output, including clinical decision recommendations, such as diagnosis, treatment plans, and any other clinical decisions carried out by clinicians. The CDS system 110 may aid the clinician in making clinical decisions by processing and analyzing large amounts of physiological data from the monitoring devices 202. The output of the CDS system 110 may be displayed on the user device 108 via the user interface 218 in some examples.

The user device 108 may display physiological data that is received by the CDS system 110. For example, the monitoring devices 202 may communicate directly with the CDS system. The CDS system 110 may then provide data received by the monitoring devices 202 to the user device 108. In this way, the user device 108 may visually show data being processed by the CDS system in real-time or near real-time. Additionally, the CDS system 110 may monitor signal quality (e.g., signal quality index) of the signals containing the physiological data from the monitoring devices 202. Upon signal quality being degraded, including complete loss of signal, the CDS system 110 may notify the user device 108 which may display an alert to the clinician 104. In this way, in contrast with previous patient monitoring systems, status of the signal quality may be viewable by the clinician 104, allowing the clinician 104 to correct positioning of the monitoring devices more promptly.

In some examples, the user device 108 may additionally regulate communication between the monitoring devices 202 and the CDS system 110. For example, the monitoring devices 202 and the CDS system 110 may be communicatively coupled via the user device 108 such that the user device 108 may selectively pass data received from the monitoring devices 202 to the CDS system 110 depending on states of the monitoring devices 202 (e.g., positioned incorrectly or correctly). In this way, the user device 108 may interrupt data consumption of the CDS system 110, for example when the user device 108 determines that the data is not accurate due to the monitoring devices 202 being positioned incorrectly. In such examples, when the data consumption is paused or otherwise excluded form analysis by the CDs system 110, the user device 108 may not pause displaying data. Data may be viewable on a display screen of the device 108 even while not being consumed by the CDS system such that monitoring of the patient 102 by the clinician may not be interrupted. The user device 108 may send signals to the CDS system 110 to temporarily pause and to resume consuming data from the monitoring devices 202. In this way, the user device 108 may regulate data consumption of the CDS system 110 from the monitoring devices 202 to increase accuracy of data used in algorithms implemented by the CDS system 110.

The user device 108 may be a wireless computing device such as a mobile device, smartphone, tablet, etc. The user device 108 may include processor 206, memory 208, and user interface 218. The processor 206 may execute instructions stored in memory 208. The memory 208 may include random access memory, cache memories, non-volatile memories, backup memories, mass storage memory, read-only memories, and the like. The memory 208 may also include memory located physically separate from the device 108 such as any storage device in communication with the user device 108, cloud storage, etc.

An application 210 may be stored in the memory 208. The application 210 may include code that when executed by the processor 206 displays an alert for the clinician 104 when the monitoring devices 202 are misplaced. In some examples, the application 210 may additionally include code that when executed by the processor 206 reduces amounts of inaccurate data consumed by the CDS system 110. For example, the code may be executed to control data consumption of the CDS system 110 according to whether the monitoring devices 202 are misplaced according to signal quality (e.g., with degradation of signal quality or complete loss of signal indicating misplacement).

The application 210 may optionally include a locator module 212. The locator module 212 may not be included in examples where the CDS system 110 receives data directly from the monitoring devices 202 and monitors signal quality, as described above. Either the locator module 212 or the CDS system 110 may monitor quality of signals from the monitoring devices. When included, the locator module 212 may use signals received from the monitoring devices 202 to determine whether the monitoring devices 202 have been misplaced, for example fallen off the patient or otherwise moved from the tagged anatomical locations input by the clinician 104 in the reference image (e.g., reference image 1000 of FIG. 10). For example, the locator module 212 may determine the monitoring devices 202 are misplaced if the signal quality index is degraded or below a threshold, including complete loss of signals. Additionally or alternatively, in examples where one or more of the monitoring devices 202 are capable of detecting movement in addition to the physiological parameters, the monitoring devices 202 may send signals to the user device specifically indicating misplacement of the monitoring devices 202. The locator module 212 may additionally or alternatively employ other methods for detecting misplacement of the monitoring devices 202 from the tagged anatomical locations pre-selected by the clinician 104 in the reference image.

The application may include a notification module 214 which may generate an alert in response to detection of misplacement of one or more of the monitoring devices 202 (e.g., by the CDS system 110 or the locator module 212). The alert may be provided to the clinician in visual, audio, movement and/or other means. For example, a display screen on the user interface 218 may change upon detection of misplacement of the monitoring devices 202. Additionally or alternatively, for example, the user device 108 may emit a sound and/or vibrate.

The data transmission module 216 may be optionally included in the application 210 to regulate consumption of data from the monitoring devices 202 by the CDS system 110, as described above. In examples where the monitoring devices 202 communicate with the CDS system 110 via the network 106, the user device 108 may signal to the CDS system 110 via the network 106 to stop consuming data from the monitoring devices 202 while continuing to display data viewable by the clinician 104. Security measures such as data encryption, intrusion detection and prevention systems, and the like may be implemented to protect communication between the CDs system 110 and the user device 108 over the network 106. In examples where the data from the monitoring devices 202 is sent to the user device 108 and the user device subsequently sends the data to the CDS system 110, the user device 108 may stop sending at least some of the data to the network 106 and therefore pause data consumption of the CDS system 110. In some examples, the CDS system 110 may continue consuming data from some but not all of the monitoring devices 202 when it is detected which one(s) of the monitoring devices 202 are misplaced. For example, the CDS system 110 may continue consuming data from monitoring devices 202 that are not misplaced. In this way, the CDS system 110 may continue to perform analysis on data pertaining to the monitoring devices 202 in the tagged anatomical locations without disruption due to the other monitoring devices 202 not being in the tagged anatomical locations. Additionally, bandwidth and computing resource demands may be reduced by pausing data consumption for at least some of the monitoring devices 202 when one or more of the monitoring devices 202 are misplaced from their respective tagged anatomical locations.

The locator module 212, the notification module 214, and the data transmission module 216 may operate automatically such that user input is not demanded to identify misplacement of the monitoring devices 202 according to degraded signal quality or signal loss, alert the clinician 104, or pause data consumption by the CDS system 110.

Turning to FIG. 3, the system 100 is shown schematically in a second state where the monitoring devices 202 are misplaced such that one or more of the monitoring devices 202 are located in anatomical locations other than their respective tagged anatomical locations pre-selected by the clinician 104. In response, the application 210 may, in some examples, automatically pause communication between the monitoring devices 202 and the CDS system 110, as described above. For example, the communication pathway between the CDS system 110 and the monitoring devices 202 may be disrupted by the application such that at least some of the data collected by the monitoring devices 202 is not transmitted to the CDS system 110. In this way, in such examples, the CDS may not consume data from monitoring devices that are not positioned correctly (e.g., in the tagged location). Therefore, accuracy of the data received by the CDS system may be increased, thereby increasing likelihood of suitable clinical decision recommendations according to the state of the patient as represented by the physiological data obtained from the monitoring devices 202. Further, the CDS system 110 may not provide an output (e.g., clinical decision recommendations) to the user device 108 via the user interface 218 while the monitoring devices 202 are misplaced. Thus, the application of the present disclosure may increase accuracy of inputs to the CDS and decrease computing resource demand by automatically pausing data consumption in response to monitoring device(s) being misplaced in locations other than the tagged locations selected by the clinician. Additionally or alternatively, the user device 108 may alert the clinician 104 of the loss or degradation of signals, allowing more rapid response of the clinician 104 to reposition the monitoring devices 202 and therefore increased accuracy of the inputs and outputs of the CDs system.

Turning to FIG. 6, another example of the patient monitoring system 100 is shown, where the CDS system 110 further includes a CDS device 622 comprising a user interface 624 (e.g., display screen). The clinician 104 may provide input to the CDS system 110 via the user interface 624. For example, the clinician 104 may request certain algorithms of the CDS system to be executed. Additionally, the clinician 104 may view output of the CDS system 110 via the user interface 624. The CDS system 110 may not send outputs via the network 106 in such examples, and instead may display the outputs directly on the user interface 624 for the clinician 104 to view. Thus, in some examples, the clinician may utilize separate devices for CDS input/outputs and tagging input/outputs. In some of such examples, in response to monitoring devices being misplaced (e.g., upon detection by the locator module 212), an alert generated by the application 210 may be displayed on both the user interface 624 and the user interface 218. The user device 108 may communicate with the CDS device 622 via the network 106 in order to automatically pause consumption of the physiological data from the monitoring devices 202 by the CDS system 110 when the monitoring devices 202 are misplaced from the tagged anatomical locations. Additionally, the user device 108 may communicate with the CDS device 622 via the network 106 in order to automatically resume consumption of the physiological data from the monitoring devices 202 by the CDS system 110 when the monitoring devices 202 are placed in the tagged anatomical locations

Turning to FIG. 4, a flowchart of a method 400 is shown for a clinician monitoring a patient by employing an application in accordance with the present disclosure. The method 400 may be a workflow for the clinician including operation of the application in order to increase accuracy of CDS system inputs, thereby increasing quality of care for patients. In other words, the method 400 may be implemented by a clinician (e.g., the clinician 104 of FIGS. 1 and 2) when monitoring a patient (e.g., the patient of FIGS. 1 and 2) using monitoring devices (e.g., the monitoring devices 112, 114, 116 of FIG. 1 and the monitoring device 202 of FIG. 2) in order to make clinical decisions with aid from a CDS system (e.g., the CDS system 110 of FIGS. 1 and 2).

The method 400 begins at 402, wherein the clinician places a monitoring device adapted to measure a parameter on the patient at a desired anatomical location. The clinician may place one or more monitoring devices in one or more anatomical locations, where (in the case of two or more monitoring devices) the monitoring devices may measure the same parameter or a combination of parameters. For example, referencing FIG. 1, the clinician 104 may position the first monitoring device 112, the second monitoring device 114, and the third monitoring device 116 at the first anatomical location 122, the second anatomical location 124, and the third anatomical location 126, respectively. The monitoring device(s) placed at 402 may measure physiological parameters such as blood oxygen saturation, heart rate, end-tidal carbon dioxide, fraction of inspired oxygen, and any other measurable physiological parameters of the patient. The physiological parameters may include parameters that vary according to anatomical location in some patient conditions, as described above, for diagnosing such conditions with algorithms using the anatomical locations as inputs. Additionally, the physiological parameters may include parameters that do not vary within a patient, such as heart rate, which may not be used in algorithms using anatomical locations as inputs, but may be monitored by the clinician and made available to the application to provide an alert to the clinician of signal quality degradation or loss related to the location independent parameter, as described further with regards to FIG. 5. The clinician may select the corresponding anatomical locations for the monitoring devices according to where the monitoring device may be most accurate. For example, blood oxygen saturation may be measured more accurately from the ear than the finger and therefore the clinician may place a blood oxygen saturation monitoring device on the ear of the patient. Additionally, the monitoring devices may be placed according to a condition or prior condition of the patient. For example, a blood pressure monitoring device may not be placed on an arm where an intravenous line is inserted and may not be placed on an arm on the same side (e.g., patient's right or left) that has undergone surgery. Additionally, the monitoring devices may be positioned according to a suspected diagnosis. For example, to make a cardiovascular-related diagnosis, a blood pressure monitoring device may be placed on each of the right and left arm of the patient to assess a difference in blood pressure therebetween. The clinician may additionally or alternatively decide the anatomical location for each monitoring device based upon other guidelines not described above, as well as clinical knowledge and experience.

The method 400 proceeds to 404, wherein the clinician tags the physiological parameter with the anatomical location in a reference image, such as the reference image 1000 of FIG. 10. For example, the clinician may take a photo with a device such as the user device 108 of FIGS. 1 and 2 on which an application of the present disclosure is installed. Alternatively, the clinician may take a photo on another device in communication with the user device and send the photo to the user device. The reference image may include the patient with the monitoring devices tagged in the desired anatomical locations. The clinician may interact with the user interface of the user device (e.g., user interface 218 of FIG. 2) in order to tag the anatomical locations of the patient in the image with the parameters being monitored by the monitoring device. For example, the clinician may "drag and drop" an icon of a monitoring device type to the anatomical location on the patient. Alternatively, the clinician may type in input boxes or select from predetermined lists of anatomical location options to input monitoring devices with their corresponding tagged anatomical locations. Tagging may be performed by the clinician in other formats of the user interface without departing from the scope of the present disclosure. The reference image may contain the photo of the patient taken by the clinician as well as the tagged anatomical location(s) of the monitoring device(s).

The method 400 proceeds to 406, wherein the clinician saves the reference image and confirms the tagged location prior to collecting data from the sensors. In this way, the clinician may ensure the tagged location in the reference image is the same as the actual location on the patient. Therefore, physiological data gathered by the monitoring devices may be connected to the tagged anatomical locations, allowing for more accurately informed analysis by the CDS system.

The method 400 proceeds to 408, wherein the clinician implements the application to monitor the parameter(s) and request a CDS output based on the parameter(s) and the tagged anatomical locations. For example, the clinician may indicate (e.g., via a user interface of the device on which the application is implemented) to the CDS system which algorithms to run for a specific potential diagnosis. Monitoring the parameters may include the clinician observing the user interface to periodically check on the patient's status and receiving alerts in response to the monitoring devices' placement changing. Requesting the CDS output may include the clinician specifying algorithms of the CDS to execute with the physiological parameters from the monitoring devices and the tagged anatomical locations as inputs to produce outputs including clinical decision recommendations. Additionally or alternatively to manual input by the clinician, the request for CDS output may be executed automatically according to the available data, including the physiological data gathered by the monitoring devices and the tagged anatomical locations. Thus, based on the location data that is made available to the CDS system due to the reference image saved by the application, the CDS system may execute algorithms that take location data into consideration, increasing the accuracy of the CDS system algorithms.

The method 400 proceeds to 410, wherein the clinician optionally retags the parameter with a new anatomical location. For example, the monitoring device may be repositioned intentionally, removed, replaced, or an additional monitoring device may be placed on the patient. In such examples, 410 may be performed to create a new reference image reflecting the changes in anatomical locations of the monitoring devices.

As such, 410 includes 412, wherein the clinician repositions the monitoring device in the new anatomical location on the patient. There may be one or more monitoring devices positioned at one or more anatomical locations, as described above.

Additionally, 410 includes 414, wherein the clinician takes the new reference image, including a photograph or abstracted physical representation of the patient with the monitoring device in the new anatomical location, and tags the physiological parameter with the new anatomical location. In this way, the clinician may update the reference image as desired when moving monitoring devices. Retagging at 410 may be repeated more than once in examples where the clinician decides to change monitoring device locations more than once. In response to receiving the new reference image or input confirming the tagged anatomical locations, the application signals to the CDS system that the locations are updated and the CDS system algorithms may consume the updated location data accordingly. Further, in some examples where data transmission was paused, the application may also automatically resume CDS consumption of the physiological data with the new tagged anatomical locations as inputs.

The method 400 proceeds to 416, wherein the clinician views the CDS output. The CDS output may be a result of requesting CDS output at 408, where the CDS output is generated by running algorithms of the CDS system with the physiological data from the monitoring devices and their tagged anatomical locations as inputs.

The method 400 ends. After the method 400, the clinician may have access to the CDS output based upon physiological data gathered by the monitoring devices. Due to the implementation of the application, the accuracy of the data being input to the CDS may be increased. For example, the application may reduce inaccurate data input to the CDS, where the inaccurate data is physiological data gathered by the monitoring devices when in a position other than the tagged anatomical location. Thus, a quality of the CDS output (e.g., correspondence to the patient's actual condition) may be increased, thereby increasing usefulness of the CDS output in making clinical decisions.

Turning to FIG. 5, a flowchart is shown of a method 500 that is executed by a device in response to user input such as a clinician performing steps of the method 400 in FIG. 4. The method 500 may be implemented by a user device with an application in accordance with the present disclosure installed on the device (e.g., stored in memory of the device). For example, referencing FIG. 2, the processor 206 may execute the method 500 according to the application 210 stored in memory 208 of the user device 108.

The method 500 begins at 502, wherein a reference image is received and stored in memory of the device. The reference image may be inputted by the clinician, for example via steps of the method 400 in FIG. 4. The reference image may include a photograph or abstracted physical representation including a patient, one or more monitoring devices, and tagged locations of the one or more monitoring devices. For example, the reference image may resemble the exemplary reference image 1000 of FIG. 10.

The method 500 proceeds to 504, wherein physiological data collected by the monitoring device is displayed and the tagged anatomical locations are provided to a CDS system. Displaying the physiological data may include providing a display screen such as the display screen examples shown schematically in FIGS. 7-9 on a user interface of the device for the clinician to view. Providing the tagged anatomical locations to the CDS system may include passing data received by the device in the reference image to the CDS system or allowing a direct connection between the monitoring device and the CDS system to feed the data as inputs to the CDS system as described with regards to FIGS. 2 and 3. In some examples the user device may also provide physiological data to the CDS system. Signals may be sent from the monitoring devices to the user device and the CDS system (e.g., via a network such as the network 106 of FIGS. 1-3 and 6) with information including physiological data and corresponding tagged locations, as well as a signal quality index of the signals.

The method 500 proceeds to 506, wherein quality of the physiological data signal from the monitoring device is optionally monitored by the application. For example, the locator module 212 may receive signals from the monitoring devices 202 and evaluate the signal quality based on the signal quality index to detect occurrence of degradation of signal quality. For example, if the signal quality index is below a threshold, the signal quality may be considered degraded. Degradation of the signal quality, including entire loss of signals or excess noise, may indicate misplacement or other issues with the monitoring devices. In some alternative examples, the CDS system may monitor signal quality index and notify the application of issues, rather than the application monitoring the signal quality. In such examples, 506 may not be included.

The method 500 proceeds to 508, wherein it is determined whether the monitoring device is misplaced. For example, during monitoring of the physiological data quality at 506 if included, if the physiological data quality is degraded, temporarily interrupted, or lost (e.g., as detected by the application), it may be determined that the monitoring device is misplaced. Alternatively, the CDS system may detect that the quality is degraded, or the signal is temporarily interrupted or lost, and notify the user device. Additional indications of monitoring device misplacement may be considered additionally or alternatively to signal quality. For example, some monitoring devices may be able to detect movement of the monitoring device itself, indicating misplacement. As another example, physiological data changing at rapid rate may indicate misplacement. As another example, alerts generated by the monitoring devices due to low signal quality, lack of signal, noise, and the like may be observed. Other methods for detecting misplacement of the monitoring devices are possible without departing from the scope of the present disclosure.

If the monitoring device is misplaced (YES at 508), the method 500 proceeds to 510, wherein physiological data measured by the monitoring devices is automatically excluded from analysis by the CDS system. For example, the application may signal to the CDS to omit sections of data collected during a period where the monitoring devices are misplaced. As an example, the application may label the data such that when the CDS system receives data with the label, the CDS system recognizes the data is from misplaced monitoring device (e.g., of low signal quality) and therefore such data may be skipped over during analysis (e.g., execution of CDS system algorithms) to increase accuracy of the CDS system.

In some examples, 510 includes 511, wherein collection of physiological data is optionally automatically paused as an alternative to labeling. For example, transmission of physiological data from the monitoring devices to the CDS system either directly or indirectly (e.g., via the user device) may be temporarily stopped, alternatively to labeling the data as described above. In this way the quality of physiological data (e.g., accuracy) consumed by the CDS system may be increased by decreasing amounts of inaccurate physiological data fed as inputs to the CDS system. Further, computing resources may be decreased by pausing at least some inputs to the CDS system. Further still, pausing signals to the CDS system may reduce network traffic and conserve bandwidth. In examples where the application does not include the data transmission module, 510 may not be completed. In such examples, the CDS system may receive data directly and continuously from the monitoring devices.

Following 510, the method 500 proceeds to 512, wherein an alert that the monitoring device is misplaced is automatically displayed for the clinician to view. Displaying the alert may include updating a display screen to indicate an alert, making a sound associated with alerts, etc. in order to bring attention of the clinician to the misplacement of the monitoring devices. The alert may prompt the clinician to return to physical proximity of the patient to correct the positioning of the monitoring devices and/or update the reference image, as described above with regards to FIG. 4.

Following 512, the method 500 proceeds to 514, wherein the reference image is optionally replaced with a new reference image, including new tagged anatomical locations. For example, if the clinician moves the monitoring devices to new locations intentionally, the clinician may take a new photograph (or generate an abstracted physical representation) of the patient and retag the physiological parameters measured by the monitoring devices to the new anatomical locations where the monitoring parameters are positioned on the patient as described with regards to FIG. 4.

Following 514, the method 500 proceeds to 516, wherein analysis of physiological data is optionally resumed. For example, 516 may be completed depending on 510. That is, some examples include both 510 and 516, while other examples include neither 510 nor 516. Analysis of physiological data may be resumed by discontinuing labeling of the data as of low quality signals. Whether or not the reference image is replaced, the clinician may directly input confirmation to the user device that the reference image (e.g., reference image received at 502 or new reference image updated at 514) includes the correct tagged anatomical locations for each of the one or more monitoring devices. In response to confirmation from the clinician, the CDS system may include all physiological data received thereafter in analysis until signals are lost or degraded again.

In some examples, 516 may include 517, wherein collection of data from the monitoring device is optionally resumed, if paused at 511. Examples where 511 is not included may not include 516. In response to confirmation from the clinician, collection of data from the monitoring devices may be resumed. For example, the user device may signal to the CDS system to resume consuming data from the monitoring devices upon receipt of user input including the clinician confirming the tagged anatomical locations. Additionally or alternatively, the user device may reopen communication between the CDS system and the monitoring devices by activating data transmission from the monitoring devices to the CDS system via the user device.

Following 516, the method 500 returns to 508. In this way, monitoring of the locations of the monitoring devices may be iteratively repeated over the duration of patient monitoring, continuously checking the positions of the devices to ensure intended positioning.

Alternatively, if the monitoring device is not misplaced (NO at 508), the method 500 proceeds to 518, wherein, collection of data from the monitoring devices is continued. For example, the data may continue being consumed by the CDS system. In this way, accurate information as to the location of the monitoring devices may be allowed to be fed as inputs to the CDS system algorithms for producing clinical decision recommendations.

Following 518, the method 500 proceeds to 520, wherein it is determined whether analysis of the data by the CDS system is complete. For example, if the CDS system has completed execution of algorithms requested by the clinician via the user device or CDS device, it may be determined that the analysis is complete. That is, if the CDS system has produced an output, the analysis of the physiological data and tagged anatomical locations may be complete.

If the analysis is complete (NO at 520), the method 500 returns to 508. In this way, the device may continue monitoring the placement of the monitoring devices throughout data collection up until completion of the requested CDS algorithms, which may be requested by user input and/or automatically as described above.

Alternatively, if the analysis is complete (YES at 520), the method 500 proceeds to 522, wherein results of the analysis are displayed. For example, the results may be displayed on the user interface of the device for the clinician to view. Alternatively, the clinician may view the results on a separate device such as the CDS device 622 of FIG. 6. The method 500 ends after 522.

Upon completion of the method 500, the clinician may make an informed clinical decision based upon the monitored physiological parameters and the CDS system output. Due to the application of the present disclosure monitoring positioning of the monitoring devices and ensuring the clinician is aware of and able to rectify misplaced monitoring devices by alerting the clinician, the physiological data input to the CDS system and therefore the CDS output considered by the clinician may be more accurate. Thus, quality of clinical decisions based at least in part upon the physiological data and/or the CDS system may be increased. For example, higher quality clinical decisions may include more accurate diagnosis as to the patient's condition, more effective treatment plans leading to increased success in patient outcomes, etc. Further, by automatically pausing data consumption of the CDS system, the application of the present disclosure may decrease computing resources and conserve bandwidth in addition to increasing quality (e.g., accuracy with regards to the actual patient condition and effectiveness of treatments in increasing a patient's health or quality of life) of outputs of the CDS.

Turning to FIG. 11, a signal diagram 1100 with exemplary signals between the CDS system 110, the monitoring device 202, and the user device 108 is shown.

A first signal 1102 may be sent from the monitoring device 202 to the CDS system. The first signal 1102 may include physiological parameter data measured from a patient (e.g., the patient 102 of FIGS. 1-3 and 6), as well as signal quality metrics including signal quality index. The first signal 1102 may be sent continuously such that the CDS system 110 monitors the physiological data and signal quality of the first signal 1102 continuously.

A second signal 1104 may be sent from the user device 108 to the CDS system 110. The second signal 1104 may include tagged anatomical locations according to the physiological parameter measured by the monitoring device 202. The tagged anatomical locations may be inputted by a clinician (e.g., the clinician 104 of FIGS. 1-3 and 6) prior to the second signal 1104 being transmitted. For example, the clinician may input a reference image such as the reference image 1000 of FIG. 10 into the application of the present disclosure on the user device 108. In response to receiving the input from the clinician, the user device may transmit the second signal 1104.

A third signal 1106 may be sent continuously from the CDS system 110 to the user device 108. The third signal 1106 may include the physiological data measured by the monitoring device 202 and provided to the CDS system 110 via the first signal 1102. The user device 108 may display the physiological data such that the data is visible to the clinician, as described further below. In alternative examples, the user device 108 may instead receive signals directly from the monitoring device 202 and provide the physiological data to the CDS system 110. In such examples, the user device 108 may monitor the signal quality from the monitoring device 202, rather than the CDS system 110. Additionally, in such examples, the user device 108 may regulate (e.g., initiate, pause, etc.) data transmission to the CDS system 110 to exclude data gathered from the monitoring device 202 when the monitoring device is misplaced, according to signal quality.

A fourth signal 1108 may be sent from the CDS system 110 to the user device 108. The fourth signal 1108 may notify the user device of loss or degradation of the first signal 1102. As such, the fourth signal 1108 may be sent in response to the CDS system 110 detecting loss or degradation of quality of the first signal 1102. The user device 108 may, in response to receiving the fourth signal 1108, display an alert visible to the clinician that the first signal 1102 was degraded or lost, potentially indicating misplacement of the monitoring device 202. Additionally, in response to receiving the fourth signal 1108, the application on the user device 108 may label data collected from the monitoring device 202 so as to be excluded by the CDS system 110 in analysis.

For example, to label the data, a fifth signal 1110 sent from the user device 108 to the CDS system 110 may include a label for the physiological location indicating the data is of poor quality, rather than a physiological location tag. In this way, the user device 108 may inform the CDS system 110 that the data is to be excluded from analysis. The clinician may then correct the misplacement or other issue with the monitoring device 202 that affects the signal. In other examples where the user device 108 receives data directly from the monitoring devices and relays the information to the CDS system 110, the user device 108 may pause transmission of data to the CDS system 110 in response to detecting degradation or loss of signal from the monitoring device 202 to exclude such data from analysis by the CDS system 110, rather than label the data.

In this way, the clinician may view the physiological data remotely via the user device 108, and be notified of misplacement of the monitoring device 202 upon detection, leading to prompt correction to restore the signal quality. Further, in at least some examples, data collected by the monitoring device 202 may be excluded from analysis by the CDS system 110 in response to signals containing the physiological data being degraded or lost, increasing accuracy of outputs (e.g., diagnosis recommendations) from the CDS system 110.

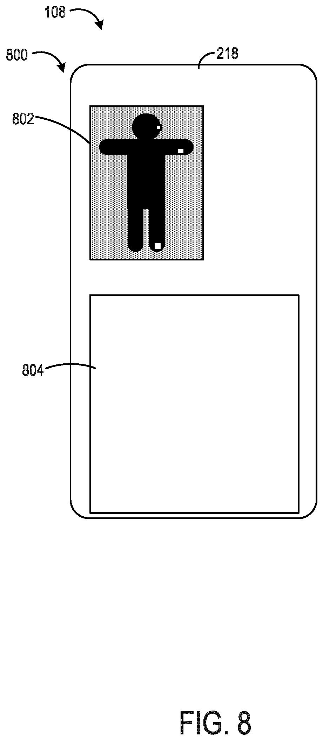

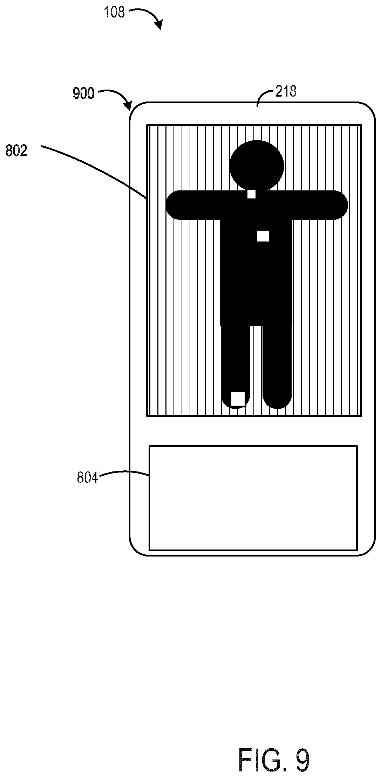

Turning to FIG. 7, an example of the user interface 218 is shown in a monitoring state where physiological data gathered by monitoring devices is displayed on a display screen 700 of the device 108. The display screen 700 may include one or more sections, each section representing a different patient. For example, a first section 702 shows data for a first patient, and a second section 704 shows data for a second patient. In this way, a clinician (e.g., clinician 104) viewing the display screen 700 may monitor more than one patient concurrently. Additionally, showing the reference image including a photograph or abstract physical representation, such as a cartoon indicating age and gender, may increase situational awareness of the clinician, as described further below in regards to FIGS. 8 and 9.

The physiological data may be displayed in different formats. For example, the first section 702 shows the physiological data of the first patient in a first format 706 comprising blocks where current readings from the monitoring devices are shown as numbers. Additionally, the first format 706 may include an alert box 710. The alert box 710 may flash, change colors, or otherwise change in order to bring attention of the clinician to an alert such as a notification of monitoring device misplacement, CDS system analysis completion, physiological parameters outside of a desired range or changing rapidly, and other notifications regarding a state of the patient and the monitoring devices. The second section 704 shows the physiological data of the second patient in a second format 708 comprising timelines showing changes in the physiological data over a selected time period (e.g., 10 minutes, 30 minutes, 2 hours, or any other amount of time) in line graphs. The second format 708 may also include the alert box 710. In this way, by using the application of the present disclosure stored in memory of the device 108, the clinician may monitor patients remotely and be alerted via visual alerts of misplacement of monitoring devices and other notable events pertaining to the patient's condition according to the physiological data, the tagged anatomical locations, and the signal quality index.

Turning to FIGS. 8 and 9, additional examples of a display screen are shown for when the monitoring devices are positioned correctly and incorrectly, respectively. Specifically, display screen 800 in FIG. 8 shows an exemplary display screen that may be displayed while the monitoring devices are physically in the anatomical locations that match the tagged anatomical locations in the reference image input to the device 108 by the clinician via the user interface 218. The display screen 800 may include a reference image section 802 and a physiological data section 804. The reference image section 802 may display the reference image (e.g., reference image 1000 of FIG. 10) inputted to the device 108 by the clinician. The reference image section 802 may change when monitoring devices are misplaced. For example, the reference image section 802 may display live updates in the locations of the monitoring devices such that the clinician may view the monitoring device locations in real time or near real-time. The physiological data section 804 may show physiological data measured by the monitoring devices in real-time or near real-time, such as in the first format 706 or the second format 708 of FIG. 7. Display screen 900 in FIG. 9 shows an exemplary display screen that may be displayed in response to detection of misplacement of the monitoring devices. For example, the locator module 212 of FIGS. 2, 3, and 6 may detect misplacement of the monitoring devices (e.g., according to signal quality index) and automatically initiate a change in the display screen from display screen 800 to display screen 900. The display screen 900 may indicate an alert via various visual changes from the display screen 800. The reference image section 802 may show monitoring devices in locations other than the tagged anatomical locations input by the clinician. Additionally, the reference image section may change color between display screen 800 and display screen 900 to emphasize the misplacement of monitoring devices. Additionally, reference image section 802 may be enlarged in display screen 900 compared to display screen 800 to further draw attention to the misplacement of the monitoring devices. Additionally, sounds and/or movement such as vibration of the device may be utilized to alert the clinician of the misplacement of the monitoring devices from their tagged anatomical locations. In this way, the clinician may monitor the patient remotely and be alerted as to movement of the monitoring devices from the tagged anatomical locations so as to correct the positioning more quickly. Thus, less data may be measured by the monitoring devices from the wrong anatomical locations of the patient. Further, concurrently with changing from display screen 800 to display screen 900 in response to detecting monitoring device misplacement, the application of the present disclosure may, in some examples, automatically exclude data from analysis by the CDS system (e.g., automatically pause data consumption by the CDS system), thereby reducing data from the wrong anatomical locations being fed into the CDS system. Reducing data transmission from monitoring devices in the wrong anatomical locations to the CDS system may reduce computing resource demand, conserve bandwidth, and result in increased quality of CDS system outputs. Alternatively, the data measured during periods where the monitoring devices are misplaced may be labeled as low quality and excluded from analysis by the CDs algorithms, thereby increasing accuracy of the CDS output.

The technical effect of anatomical location tagging with the application in accordance with the present disclosure is to increase accuracy of physiological parameter data and location data collected throughout the period of patient monitoring so as to increase accuracy of outputs (e.g., diagnosis) of the CDS system. By automatically excluding data from analysis by the CDS system (e.g., pausing consumption of data from the monitoring devices by the CDS system) in response to automatically detecting misplacement of the monitoring devices, amounts of inaccurate data being analyzed by the CDS system may be decreased. Therefore, clinicians implementing the application of the present disclosure to tag monitoring devices to the anatomical locations may increase quality of care of patients by increasing accuracy of information analyzed by the clinician and the CDS system consuming physiological data generated by the monitoring devices. Further, in some examples, computing resource demand may be reduced due to decreased data transmission when the monitoring devices are misplaced. For example, when the application automatically pauses consuming data that would otherwise be collected, overall data sent to the CDS system may be decreased. In other words, automatically pausing data consumption by the CDS system in response to monitoring devices being misplaced relative to the tagged anatomical locations in the reference image may decrease amounts of inaccurate data being analyzed by the CDS system. For example, in other systems incapable of automatically pausing and resuming data consumption, a clinician noticing a misplaced monitoring device after partial or full analysis of a CDS system may result in less accurate outputs, and in some cases demand for restarting the CDS system analysis to ensure the data analyzed is from the correct anatomical locations. In such an example, computing power may be increased due to restarting the analysis. Hence, systems and/or methods of the present disclosure may reduce an amount of data processed by the CDS system to produce an output, compared to other systems. In this way, computing efficiency of the CDS system may be increased.

The disclosure also provides support for a method, comprising: receiving a reference image including an image of a monitoring device, where the monitoring device is adapted to measure physiological data of a patient tagged to an anatomical location of the patient, displaying the physiological data from the monitoring device and providing the tagged anatomical location to a clinical decisions support (CDS) system, monitoring signal quality index of signals from the monitoring device, and in response to the monitoring device being misplaced, automatically pausing analysis of the physiological data by the CDS system and displaying an alert that the monitoring device is misplaced. In a first example of the method, the method further comprises: replacing the reference image with a new reference image including a new tagged anatomical location following automatically pausing. In a second example of the method, optionally including the first example, the method further comprises: resuming analysis of the physiological data from the monitoring device in response to user input following automatically pausing analysis of the physiological data by the CDS system and displaying the alert that the monitoring device is misplaced. In a third example of the method, optionally including one or both of the first and second examples, the user input includes confirmation that the tagged anatomical location is correct, where the user input is input by a clinician via a user interface. In a fourth example of the method, optionally including one or more or each of the first through third examples, the reference image further includes an image of the patient with the monitoring device positioned at the tagged anatomical location of the patient, and wherein the reference image is a photograph or abstracted physical representation. In a fifth example of the method, optionally including one or more or each of the first through fourth examples, the method further comprises detecting the monitoring device is misplaced according to the signal quality index or loss of signal prior to automatically pausing analysis of the physiological data by the CDS system and displaying the alert that the monitoring device is misplaced. In a sixth example of the method, optionally including one or more or each of the first through fifth examples, the method further comprises displaying the reference image.

The disclosure also provides support for a method, comprising: placing a monitoring device adapted to measure a physiological parameter on a patient at an anatomical location, tagging the physiological parameter with the anatomical location in a reference image via a user interface of a user device in communication with the monitoring device, saving the reference image in memory of the user device, and implementing an application to view the reference image and monitor the physiological parameter and the anatomical location. In a first example of the method, implementing the application comprises: displaying physiological data from the monitoring device and providing the tagged anatomical location and a signal quality index to a clinical decisions support (CDS) system, displaying the physiological data and monitoring the signal quality index on the user interface, and in response to the monitoring device being misplaced, automatically excluding physiological data measured by the monitoring device from analysis by the CDS system and displaying an alert that the monitoring device is misplaced. In a second example of the method, optionally including the first example, the method further comprises: retagging the physiological parameter with a new anatomical location. In a third example of the method, optionally including one or both of the first and second examples, retagging comprises repositioning the monitoring device in the new anatomical location and tagging the new anatomical location in a new reference image. In a fourth example of the method, optionally including one or more or each of the first through third examples, excluding the physiological data includes automatically labeling the physiological data or pausing consumption of the physiological data by the CDS system. In a fifth example of the method, optionally including one or more or each of the first through fourth examples, implementing the application further comprises resuming analysis of physiological data from the monitoring device in response to receiving user input confirmation that the tagged anatomical location is correct following automatically excluding the physiological data and displaying the alert that the monitoring device is misplaced. In a sixth example of the method, optionally including one or more or each of the first through fifth examples, implementing the application further comprises detecting that the monitoring device is misplaced by identifying the signal quality index is below a threshold or signals are lost. In a seventh example of the method, optionally including one or more or each of the first through sixth examples, the method further comprises: viewing an output of the CDS system via the user interface.

The disclosure also provides support for a patient monitoring system, comprising: a patient, a clinician, one or more monitoring devices positioned at anatomical locations of the patient and adapted to measure one or more physiological parameters, a user device including a user interface and instructions stored in memory, the instructions executable by a processor of the user device to: displaying physiological data from the one or more monitoring devices, provide the physiological data and the anatomical locations as inputs to a clinical decision support (CDS) system, monitor quality of the physiological data, and in response to detecting at least one of the one or more monitoring devices being misplaced, automatically pausing analysis of physiological data from the at least one of the one or more monitoring devices and displaying an alert on the user interface that at least one of the one or more monitoring devices is misplaced. In a first example of the system, the system further comprises a network through which the one or more monitoring devices, the user device, and the CDS system may communicate wirelessly. In a second example of the system, optionally including the first example, the instructions are further executable to resume analysis of physiological data from the one or more monitoring devices in response to user input indicating confirmation of correct tagged anatomical locations. In a third example of the system, optionally including one or both of the first and second examples, detecting at least one of the one or more monitoring devices being misplaced includes monitoring a signal quality index. In a fourth example of the system, optionally including one or more or each of the first through third examples, the user device is configured to display outputs of the CDS system.

In another representation, the disclosure also provides support for a method, comprising: receiving a reference image of a monitoring device adapted to measure physiological data of a patient tagged to an anatomical location of the patient, initiating collecting the physiological data from the monitoring device and providing the physiological data and the tagged anatomical location to a clinical decisions support (CDS) system, displaying the physiological data and monitoring signal quality index of signals from the monitoring device, and in response to the monitoring device being misplaced, automatically pausing consumption of the physiological data by the CDS system and displaying an alert that the monitoring device is misplaced. In a first example of the method, the method further comprises: replacing the reference image with a new reference image including a new tagged anatomical location following automatically pausing. In a second example of the method, optionally including the first example, the method further comprises: resuming collecting data from the monitoring device in response to user input following automatically pausing consumption of the physiological data by the CDS system and displaying the alert that the monitoring device is misplaced. In a third example of the method, optionally including one or both of the first and second examples, the user input includes confirmation that the tagged anatomical location is correct, where the user input is input by a clinician via a user interface. In a fourth example of the method, optionally including one or more or each of the first through third examples, the reference image comprises an image of the patient with the monitoring device positioned at the tagged anatomical location of the patient. In a fifth example of the method, optionally including one or more or each of the first through fourth examples, the method further comprises detecting the monitoring device is misplaced according to the signal quality index prior to automatically pausing consumption of the physiological data by the CDS system and displaying the alert that the monitoring device is misplaced. In a sixth example of the method, optionally including one or more or each of the first through fifth examples, the method further comprises displaying the reference image.

In another representation, the disclosure also provides support for a method, comprising: placing a monitoring device adapted to measure a physiological parameter on a patient at an anatomical location, tagging the physiological parameter with the anatomical location in a reference image via a user interface of a user device in communication with the monitoring device, saving the reference image in memory of the user device, and implementing an application to view the reference image and monitor the physiological parameter and the anatomical location. In a first example of the method, implementing the application comprises: initiating collecting physiological data from the monitoring device and providing the physiological data, the tagged anatomical location, and a signal quality index to a clinical decisions support (CDS) system, displaying the physiological data and monitoring the signal quality index on the user interface, and in response to the monitoring device being misplaced, automatically pausing collecting physiological data from the monitoring device and displaying an alert that the monitoring device is misplaced. In a second example of the method, optionally including the first example, the method further comprises: retagging the physiological parameter with a new anatomical location. In a third example of the method, optionally including one or both of the first and second examples, retagging comprises repositioning the monitoring device in the new anatomical location and tagging the new anatomical location in a new reference image. In a fourth example of the method, optionally including one or more or each of the first through third examples, automatically pausing collecting data from the monitoring device includes automatically pausing consumption of the physiological data by the CDS system. In a fifth example of the method, optionally including one or more or each of the first through fourth examples, implementing the application further comprises resuming collecting physiological data from the monitoring device in response to receiving user input confirmation that the tagged anatomical location is correct following automatically pausing collecting physiological data from the monitoring device and displaying the alert that the monitoring device is misplaced. In a sixth example of the method, optionally including one or more or each of the first through fifth examples, implementing the application further comprises detecting that the monitoring device is misplaced by identifying the signal quality index is below a threshold. In a seventh example of the method, optionally including one or more or each of the first through sixth examples, the method further comprises: viewing an output of the CDS system via the user interface.

In another representation, the disclosure also provides support for a patient monitoring system, comprising: a patient, a clinician, one or more monitoring devices positioned at anatomical locations of the patient and adapted to measure one or more physiological parameters, a user device including a user interface and instructions stored in memory, the instructions executable by a processor of the user device to: initiate collecting physiological data from the one or more monitoring devices, provide the physiological data and the anatomical locations as inputs to a clinical decision support (CDS) system, display the physiological data on the user interface and monitor quality of the physiological data, and in response to detecting at least one of the one or more monitoring devices being misplaced, automatically pausing collecting physiological data from the at least one of the one or more monitoring devices and displaying an alert on the user interface that at least one of the one or more monitoring devices is misplaced. In a first example of the system, the system further comprises a network through which the one or more monitoring devices, the user device, and the CDS system may communicate wirelessly. In a second example of the system, optionally including the first example, the instructions are further executable to resume collecting physiological data from the one or more monitoring devices in response to user input indicating confirmation of correct tagged anatomical locations. In a third example of the system, optionally including one or both of the first and second examples, detecting at least one of the one or more monitoring devices being misplaced includes monitoring a signal quality index. In a fourth example of the system, optionally including one or more or each of the first through third examples, the user device is configured to display outputs of the CDS system.

FIGS. 1-3 and 6-10 show example configurations with relative positioning of the various components. If shown directly contacting each other, or directly coupled, then such elements may be referred to as directly contacting or directly coupled, respectively, at least in one example. Similarly, elements shown contiguous or adjacent to one another may be contiguous or adjacent to each other, respectively, at least in one example. As an example, components laying in face-sharing contact with each other may be referred to as in face-sharing contact. As another example, elements positioned apart from each other with only a space there-between and no other components may be referred to as such, in at least one example. As yet another example, elements shown above/below one another, at opposite sides to one another, or to the left/right of one another may be referred to as such, relative to one another. Further, as shown in the figures, a topmost element or point of element may be referred to as a "top" of the component and a bottommost element or point of the element may be referred to as a "bottom" of the component, in at least one example. As used herein, top/bottom, upper/lower, above/below, may be relative to a vertical axis of the figures and used to describe positioning of elements of the figures relative to one another. As such, elements shown above other elements are positioned vertically above the other elements, in one example. As yet another example, shapes of the elements depicted within the figures may be referred to as having those shapes (e.g., such as being circular, straight, planar, curved, rounded, chamfered, angled, or the like). Further, elements shown intersecting one another may be referred to as intersecting elements or intersecting one another, in at least one example. Further still, an element shown within another element or shown outside of another element may be referred as such, in one example.

It will be appreciated that the configurations and routines disclosed herein are exemplary in nature, and that these specific embodiments are not to be considered in a limiting sense, because numerous variations are possible. Moreover, unless explicitly stated to the contrary, the terms "first," "second," "third," and the like are not intended to denote any order, position, quantity, or importance, but rather are used merely as labels to distinguish one element from another. The subject matter of the present disclosure includes all novel and non-obvious combinations and sub-combinations of the various systems and configurations, and other features, functions, and/or properties disclosed herein.

The following claims particularly point out certain combinations and sub-combinations regarded as novel and non-obvious. These claims may refer to "an" element or "a first" element or the equivalent thereof. Such claims should be understood to include incorporation of one or more such elements, neither requiring nor excluding two or more such elements. Other combinations and sub-combinations of the disclosed features, functions, elements, and/or properties may be claimed through amendment of the present claims or through presentation of new claims in this or a related application. Such claims, whether broader, narrower, equal, or different in scope to the original claims, also are regarded as included within the subject matter of the present disclosure.

## Claims

1. A method, comprising:
receiving a reference image including an image of a monitoring device, where the monitoring device is adapted to measure physiological data of a patient tagged to an anatomical location of the patient;
displaying the physiological data from the monitoring device and providing the tagged anatomical location to a clinical decisions support (CDS) system;
monitoring signal quality index of signals from the monitoring device; and
in response to the monitoring device being misplaced, automatically pausing analysis of the physiological data by the CDS system and displaying an alert that the monitoring device is misplaced.

2. The method of claim 1, further comprising replacing the reference image with a new reference image including a new tagged anatomical location following automatically pausing.

3. The method of claim 1, further comprising resuming analysis of the physiological data from the monitoring device in response to user input following automatically pausing analysis of the physiological data by the CDS system and displaying the alert that the monitoring device is misplaced.

4. The method of claim 3, wherein the user input includes confirmation that the tagged anatomical location is correct, where the user input is input by a clinician via a user interface.

5. The method of claim **1,** wherein the reference image further includes an image of the patient with the monitoring device positioned at the tagged anatomical location of the patient, and wherein the reference image is a photograph or abstracted physical representation.

6. The method of claim 1, wherein the method further comprises detecting the monitoring device is misplaced according to the signal quality index or loss of signal prior to automatically pausing analysis of the physiological data by the CDS system and displaying the alert that the monitoring device is misplaced.

7. The method of claim 1, wherein the method further comprises displaying the reference image.

8. The method of claim 1, wherein the method further comprises receiving a new reference image with a new tagged anatomical location, and providing the new tagged anatomical location to the CDS system.

9. The method of claim 1, wherein pausing analysis of the physiological data comprises automatically labeling the physiological data or pausing consumption of the physiological data by the CDS system to exclude the physiological data from analysis by the CDS system.

10. A patient monitoring system, comprising:
a patient;
a clinician;
one or more monitoring devices positioned at anatomical locations of the patient and adapted to measure one or more physiological parameters; and
a user device including a user interface and instructions stored in memory, the instructions executable by a processor of the user device to:
displaying physiological data from the one or more monitoring devices;
provide the physiological data and the anatomical locations as inputs to a clinical decision support (CDS) system;
monitor quality of the physiological data; and
in response to detecting at least one of the one or more monitoring devices being misplaced, automatically pausing analysis of physiological data from the at least one of the one or more monitoring devices and displaying an alert on the user interface that at least one of the one or more monitoring devices is misplaced.

11. The patient monitoring system of claim 10, wherein the system further comprises a network through which the one or more monitoring devices, the user device, and the CDS system may communicate wirelessly.

12. The patient monitoring system of claim 10, wherein the instructions are further executable to resume analysis of physiological data from the one or more monitoring devices in response to user input indicating confirmation of correct tagged anatomical locations.

13. The patient monitoring system of claim 10, wherein detecting at least one of the one or more monitoring devices being misplaced includes monitoring a signal quality index.

14. The patient monitoring system of claim 10, wherein the user device is configured to display outputs of the CDS system.

15. The patient monitoring system of claim 10, wherein automatically pausing analysis of the physiological data comprises automatically labeling the physiological data or pausing consumption of the physiological data by the CDS system to exclude the physiological data from analysis by the CDS system.
